# EUROPÄISCHE PATENTSCHRIFT

(11) **EP 2 101 778 B1**
(45) Veröffentlichungstag und Bekanntmachung des Hinweises auf die Patenterteilung: **13.04.2011**
(21) Anmeldenummer: 07846967.3
(22) Anmeldetag: 03.12.2007
(51) Int. Cl.: A61K 31/505, A61P 9/00

(54) **ECTOIN ZUR BEHANDLUNG VON VASCULAR LEAKS**
ECTOIN FOR TREATING VASCULAR LEAKS
ECTOINE POUR LE TRAITEMENT DE FUITES VASCULAIRES

(30) Priorität: 01.12.2006 DE 102006056766
(43) Veröffentlichungstag der Anmeldung: 23.09.2009
(73) Patentinhaber: Bitop Aktiengesellschaft Für Biotechnische Optimierung, 58453 Witten (DE)
(72) Erfinder: SCHWARZ, Thomas, 42799 Leichlingen (DE); LENTZEN, Georg, 58313 Herdecke (DE)
(74) Vertreter: Thiel, Christian
(86) Internationale Anmeldenummer: PCT/EP2007/010479
(87) Internationale Veröffentlichungsnummer: WO 2008/064916

(56) Entgegenhaltungen:
- WO-A-00/76528
- WO-A-2005/094771
- WO-A-2006/097263
- US-A1- 2004 071 691

## Beschreibung

Die Erfindung betrifft Ectoinen zur prophylaktischen oder therapeutischen Behandlung von Endothelfunktionsstörungen, nämlich vascular Leaks.

Aus extremophilen Organismen isolierte niedermolekulare organische chemische Verbindungen haben einen bemerkenswerten Einfluss auf die thermische, chemische und physikalische Stabilität von komplexen organischen Verbindungen und biologischen Zellstrukturen. Solche organische Verbindungen werden als Osmolyte oder kompatible Solute bezeichnet und haben inzwischen Eingang gefunden in zahlreiche kosmetische Präparate.

Zu den kompatiblen Soluten gehören Zucker, Schwefelverbindungen, Polyole, Aminosäuren und Aminosäurederivate, sowie Tetrahydropyrimidinderivate, wie Ectoin und Hydroxyectoin. Sie werden von extremophilen Mikroorganismen unter Stressbedingungen synthetisiert und dienen der Stabilität der Zellstrukturen dieser Mikroorganismen unter extremen thermischen, chemischen und physikalischen Bedingungen. Ein Beispiel hierfür sind halophile Mikroorganismen, die sich dem wechselnden Salzgehalt in salzhaltiger Umgebung anpassen müssen und darin überleben müssen.

Beispiele für kompatible Solute, die Einsatz in der Praxis gefunden haben, sind z. B Ectoin und Hydroxyectoin.

EP-A-0 553 884 beschreibt gereinigte Tetrahydropyrimidinderivate und diese Derivate enthaltende pharmazeutische Zusammensetzungen. Sie eignen sich zum Schutz der DNA vor Schäden durch DNA-bindende Wirkstoffe, Carzinogene, Mutagene und Strahlung.

Des Weiteren ist von Ectoin und seinen Derivaten die stabilisierende Wirkung auf Protein- und Nukleinsäurestrukturen bekannt, die zur Stabilisierung von biologischem Material und Arzneimittelzubereitungen herangezogen werden kann.

Gemäß WO-00/076528 sind Ectoin und Hydroxyectoin geeignet, die zur Effektivität Protein enthaltender Wirkstoffe zu erhöhen. Proteinhaltige Wirkstoffe in diesem Sinne sind Antikörper, und Immuntoxine. Genannt wird das Anti-CD30-Immunotoxin Ki-4.dgA.

Gemäß WO 00/076528 ist die proteinhaltige Substanz der eigentliche Wirkstoff. Kompatible Solute bzw. Ectoine werden lediglich zur Effektivitätssteigerung herangezogen, d. h. als reine Adjuvantien. In diesem Sinne ist auch die Verwendung zur Reduzierung cytotoxischer Aktivitäten, insbesondere des "vascular leak syndrome" (VLS) zu verstehen. Bei dem Vascular Leak Syndrom führt eine Endothelzellschädigung u. a. zu einem Verlust von Albumin im Intrazellularraum, was zu einer zunehmenden Ansammlung intrastitieller Flüssigkeit und zur Ausbildung vor allem von Ödemen, Hypotonien und Tachykardien führt.

Das Vascular Leak Syndrom tritt häufig im Zusammenhang mit der Freisetzung von Immuntoxinen unter Stressbedingungen auf, beispielsweise bei schwerer Sepsis und septischem Schock, aber auch im Zusammenhang mit dem Anschluss von Säuglingen und Kleinkindern an Herz-Lungenmaschinen, bei Verbrennungen und dem "systemic in flammatory response syndrome" (SIRS)

Interleukin-2 ist ein wichtiges Zytokin, das therapeutisch beispielsweise bei bestimmten Krebsformen eingesetzt wird. Der Einsatz dieses hochwirksamen Medikaments ist allerdings aufgrund der auftretenden toxischen Nebenwirkungen stark eingeschränkt. Zu nennen ist hier das Vascular Leak Syndrom, das auch bei anderen lmmuntoxinen auftritt. Als Auslöser des Vascular Leak Syndroms gilt beispielsweise auch das starke pflanzliche Gift Rizin.

Das Vascular Leak Syndrom, häufig auch als Capillary Leak Syndrom oder Kapillarleak-Syndrom bezeichnet, kann ferner bei schweren operativen Eingriffen auftreten, etwa nach Knochenmarktransplantationen, kardiopulmonären Bypass-Operationen und der hämophagocytischen Lymphohistiocytosis. Lungenödeme, wie sie z. B. beim "acute lung injury" und Acute Respiratory Distress Syndrome" (ARDS) auftreten, können ebenfalls von einem massiven Vascular Leak begleitet werden.

Typische Einzelsymptome des VLS sind beispielsweise Überdruck, Hypoalbuminemia, Lungenödeme, generelle Ödeme sowie der eigentliche vascular Leak. Ähnliche Symptome zeigen sich beim septischen Schock, SIRS, hämorrhagischen Fiebern, etwa dem Dengue-Fieber, dem ARBO-Viren-Fieber, bei Marburg- und Ebola-Infektionen wie auch anderen tropischen Fiebererkrankungen.

Ursächlich für das SIRS können Verletzungen, Verbrennungen, schwere Blutungen, Ischämie, Anaphylaxie oder auch eine hämorrhagischnekrotisierende Pankreatitis sein. Neben einem vascular Leak treten dabei stark erniedrigte oder erhöhte Körpertemperaturen, erhöhte Herzfrequenz, Tachypnoe, ggf. mit einem Sauerstoffmangel und eine erniedrigte oder erhöhte Leukozytenzahl auf. Es handelt sich um ein Krankheitsbild, dass einer Sepsis ähnelt, ohne das eine Infektion nachweisbar ist.

Bei allem beschriebenen Symptomen und Krankheitsverläufen kommt es zu einer Störung der Endothelzellbarriere, welche die entsprechenden Körperkompartimente voneinander trennt. Wird diese Barriere durch eine exogene oder endogene Noxe gestört, können Flüssigkeit und Biomoleküle aus dem vaskulären System in die umliegenden Gewebe entweichen, es kommt zu den beschriebenen Symptomen und resultierenden Krankheitsbildern.

Die Mechanismen, die zur gesteigerten Endothelzellpermeabilität führen, sind Mediator-abhängig. So wird beispielsweise die Zunahme der 11-2-bedingten Endothelzellpermeabilität auf die Induktion der Apoptose der Endothelzellen zurückgeführt. Lipopolysaccharid (LPS), ein Bestandteil der äußeren Membran gramnegativer Bakterien, vermittelt die gesteigerte Endothelzellpermeabilität durch die Modulation interendothelialer Tight/Gap Junction assoziierte Proteine, aber auch durch die Induktion einer Endothelzellapoptose. Histamin dagegen, ein sehr früher Mediator der systemischen Entzündung, bedingt eine gesteigerte Endothelzellpermeabilität in erster Linie durch die Modulation von Tight/Gap Junction Proteinen.

Insbesondere Hydroxyectoin stabilisiert die Endothelzellbarriere durch die Stabilisierung der Membranbestandteile. Durch die "preferential Exclusion" wird Hydroxyectoin aus der Hydrathülle der verschiedenen Membranbestandteile ausgeschlossen und führt somit zu einer Kompaktierung der Proteine und anderer Membranbestandteile (Lipide etc.) Diese sind nun schwächer für die Noxen erreichbar (Rezeptorebene, z.B. II-2 Rezeptor) und die nachgeschalteten Signalwege werden weniger stark aktiviert, eine Induktion Apoptose kann verhindert werden.

Diese Kompaktierung stabilisiert auch die Membranbestandteile (Protein-Komplexe) welche die Tight Junctions (die Verbindung zwischen den Endothelzellen) bilden. Diese können durch im vaskulären System auftretendes LPS oder Histamin weniger beeinflusst werden, wodurch die Verbindung zwischen den Zellen stärker erhalten bleibt. Somit wird auch bei LPS/Histamin-Belastung die Endothelzellbarriere stabilisiiert.

Entsprechend eignen sich Ectoine sowohl zum Schutz vor sowie auch zur Behandlung einer Vaskulitis und insbesondere von Endotheldysfunktionen bzw. Endothelfunktionsstörungen, im folgenden zumeist als vascular Leak oder VLS bezeichnet. Weiterhin ist auch eine Wirksamkeit bei einer Funktionsstörung der Barrierewirkung von Epithelzellen gegeben.

Es wurde gefunden, dass Ectoine, nämlich Ectoin selbst und Hydroxyectoin, als alleinige Wirkstoffe vascular Leaks entgegenwirken können. Diese Substanzen sind also zur Prophylaxe und Behandlung des VLS geeignet.

Die Erfindung betrifft entsprechend ein Mittel zur Prophylaxe und Hemmung bzw. Behandlung von vascular Leaks. Darin kommen Ectoine insbesondere als alleinige Wirkstoffe zum Einsatz.

Ectoine im Sinne der Erfindung sind (4S)-1,4,5,6-Tetrahydropyrimidin-4-carbonsäuren und ihre pharmazeutisch akzeptablen Salze.

Das Tetrahydropyrimidin kann in der 5-Position durch eine Hydroxygruppe substituiert sein, insbesondere durch ein (5S)-Hydroxygruppe. Alle Formen von vascular Leaks werden verstanden, wie sie oben im Zusammenhang mit den näher beschriebenen Erkrankungen und Krankheitserscheinungen geschildert werden. Es versteht sich, dass bei den Krankheitserscheinungen nicht das gesamte Krankheitsbild vorliegen muss; das erfindungsgemäße Mittel kann auch zur Prophylaxe oder Behandlung von einzelnen Symptomen, die im Zusammenhang mit Endothelfunktionsstörungen stehen, herangezogen werden.

Beanspruchte Ectoine sind Ectoin selbst, (4S)-2-Methyl-1,4,5,6-tetrahydropyrimidin-4-carbonsäure, und Hydroxyectoin, (4S,5S)-5-Hydroxy-2-methyl-1,4,5,6-tetrahydropyrimidin-4-carbonsäure.

Erfindungsgemäß können mehrere Ectoine zusammen eingesetzt werden. Bei dem erfindungsgemäßen Mittel kann das Ectoin selbstverständlich mit üblichen Adjuvantien und Hilfsstoffen kombiniert sein. Es kann als Tablette, Kapsel oder als Lösung zur orale oder parentiralen Verabreichung vorliegen, vorzugsweise in Form einer wässrigen Injektionslösung. Es kann in Dosen von 10 bis 10 000 mg, vorzugsweise 20 bis 1000 mg und insbesondere 50 bis 500 mg verabreicht werden.

Die erfindungsgemäß zum Einsatz kommenden Ectoine, Ectoin und Hydroxyectoin, können miteinander kombiniert werden.

Das erfindungsgemäße Mittel betrifft jede Form des Vascular Leak Syndroms, unter welchen Umständen dieses auch immer ausgelöst wird. Es betrifft insbesondere die von Toxinen und Medikamenten ausgelösten Formen von vascular Leaks, beispielsweise die durch die Verabreichung von Interleukin-2 ausgelösten Formen. Ectoine sind aber auch geeignet, der Wirkung von pflanzlichen Giften, wie Rizin, entgegenzuwirken, soweit diese das VLS auslösen. Ein weiteres Einsatzfeld ist die postoperative Prophylaxe zur Vorbeugung eines Vascular Leak Syndroms, beispielsweise bei den eingangs gegebenen Indikationen, und die Prophylaxe und Behandlung des VLS insbesondere bei Säuglingen und Kleinkindern, die mit Herz-Lungenmaschinen behandelt werden, sowie bei Hypoxie.

Ectoine im Sinne der Erfindung können beim "Retinoic Acid Syndrome" eingesetzt werden. Bei der Behandlung von akuter promyolozytischer Leukämie mit all-trans retinoic acid (ATRA-Therapie) kommt es zu teilweise sehr schweren Nebenwirkungen, die sich in einer Reihe von Symptomen wie Fieber, Gewichtszunahme, interstitiellen pulmonaren Infiltraten (VLS), pleuraler und pericardialer Effusion, episodischem Bluthochdruck und akutem Nierenversagen äußern, siehe R.S. Larson und M.S. Talman, Best Pract. Res. Clin. Haematol. 2003 Sep; 16(3):453-61.

Ein weiteres Einsatzgebiet ist das Capillary Leak Syndrome bei der Acitretin-Therapie von Psoriasis bzw. beim Auftreten des Acute Respiratory Distress Syndrome (ARDS) bei Psoriasis. Die Behandlung von Psoriasis mit Retinoic

Acid kann in seltenen Fällen das VLS mit lebensbedrohlichen Folgen auslösen, siehe M.H. Vermeer und S. Pavel, J Am. Acad. Dermatol. 2006, Oct. 20.

Als Komplikation bei pustularer und erythrodermischer Psoriasis kommt es gelegentlich zum Acute Respiratory Distress Syndrome (ARDS), das sich auf das Capillary Leak Syndrome in der Lunge zurückführen läßt, J.S. Sadeh et al., Arch. Dermatol. 1997 June, 133(6):747-50.

Es wird angenommen, dass die Schutzwirkung der Ectoine über eine Endothelzellstabilisierung abläuft, d. h. die Wirkstoffe wirken einer krankheits- oder behandlungsbedingten Vaskulitis entgegen. Es liegen Hinweise darauf vor, dass die Ectoine über die Zellmembranen wirken und einen positiven Effekt auf Entzündungserscheinungen ausüben.

Die hier geltend gemachten Wirkungen von Ectoinen werden anhand der folgenden Beispiele näher erläutert.

### Beispiel 1

### In vitro Untersuchungen zur Hemmung des Interleukin-2-induzierten VLS

Die Hemmung des II-2-verursachten VLS durch Ectoin und Hydroxyectoin wurde in einem in vitro-System mit humanen Endothelzellen (HUVEC-Zellen) untersucht. Hierzu wurden die Konzentrationen an Proleukin^{R} (Firma Chiron) ermittelt, die zur Abkugelung der HUVEC-Zellen führen. Die Abkugelung von HUVEC-Zellen gilt als Indiz für die Auslösung des VLS, siehe Baluna et al., PNAS 3957-3962, März 1999. Dieser im Lichtmikroskop sichtbare Phänotyp beruht, wie das VLS, auf der Bindung von Membranproteinen, den Integrinen.

Die Zellen wurden mit Proleukin allein oder in Kombination mit Ectoin und Hydroxyectoin inkubiert. Als Kontrollen für die Spezifität der kompatiblen Solute wurden zusätzlich Trehalose und Glukose eingesetzt.

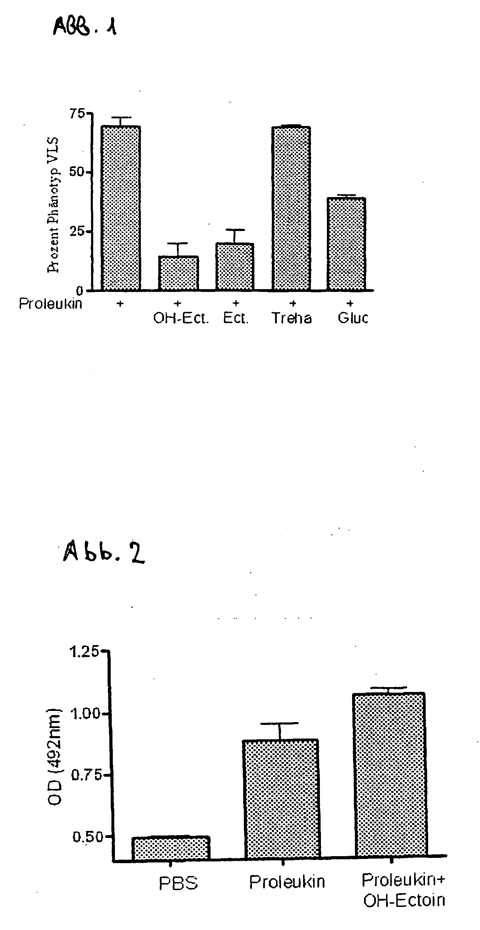

Nach 4 bis 6 Tagen wurde der Anteil normaler und abgekugelter Zellen eines Gesichtsfelds mikroskopisch bestimmt. Die Ergebnisse sind in Abbildung 1 zusammengefasst.

Abbildung 1 zeigt die prozentuale Hemmung des VLS anhand von HUVEC-Zellen nach Verabreichung von 2 mM Proleukin allein oder in Kombination mit 20 mM Hydroxyectoin (OH-Ect.), Ectoin (Ect.), Trehalose (Treha) und Glukose (Gluc).

Sowohl Hydroxyectoin als auch Ectoin vermögen den VLS-Phänotyp deutlich zu reduzieren. Ein protektiver Effekt, der nicht ganz so deutlich ist, zeigt sich ebenfalls bei Glukose, nicht aber bei Trehalose.

### Beispiel 2

Auswirkungen von Hydroxyectoin auf die Proleukin-induzierte Interferon γ-Synthese humaner Lymphozyten.

Interleukin-2, II-2, führt ebenso wie Proleukin zu einer Aktivierung von Immuneffektorzellen, die man unter anderen an einer erhöhten Interferon γ-Sekretion von NK-Zellen und T-Lymphozyten messen kann.

Hierzu wurden NK-Zellen und T-Zellen blutgesunder Spender mittels MACS-Technik (Magnetic Cell Sorting, Milteniy) isoliert. Der Nachweis der Zellen, die die Oberflächen marker CE 56, NKG2D und CD 3 exprimieren, erfolgte in FACS-Analysen.

Die Zellen wurden ohne weitere Zusätze, mit 250 U Proleukin oder mit 250 U Proleukin in Kombination mit 20 mM Hydroxyectoin für 48 Stunden inkubiert. Anschließend wurde die Konzentration von Interferon Y im Überstand der Zellen mittels ELISA bestimmt (3-fach-Bestimmungen aus zwei unabhängigen Experimenten). Die Ergebnisse zeigen, dass Hydroxyectoin die Induktion der Interferon γ-Synthese nicht hemmt, es zeigen sich im Gegenteil leicht erhöhte Werte (siehe Abbildung 2).

Die in vitro-Daten zeigen, dass die getesteten kompatiblen Solute eine Verminderung des Proleukin-induzierten VLS-Phänotyps von HUVEC-Zellen bewirken, wobei dies nicht mit einer Verminderung der Aktivität bezüglich der Interferon-Induktion von primären humanen Effektorzellen verbunden ist.

## Patentansprüche

1. Mittel zur verwendung für prophylaktische oder therapeutische Behandlung von vascular Leaks, **dadurch gekennzeichnet, dass** es Ectoin und/oder Hydroxyectoin und/oder ein pharmazeutisch annehmbares Salz davon als alleinigen Wirkstoff/alleinige Wirkstoffe enthält.

2. Mittel zur verwendung nach Anspruch 1 zur Behandlung einer Vaskulitis und insbesondere des VLS, SIRS, ARDS vascular leak syndrome, systemic inflammatory response syndrome, acute respiratory distress syndrome.

3. Mittel zur verwendung nach einem der vorstehenden Ansprüche zur Prophylaxe oder Behandlung von von Toxinen oder Medikamenten ausgelösten Formen von vascular Leaks.

4. Mittel zur verwendung nach Anspruch 3 zur Hemmung des von Interleukin-2 induziertem VLS.

5. Mittel zur verwendung nach Anspruch 1 oder 2, zur Behandlung des von hämorraghischen Fiebern ausgelöstem VLS.

6. Mittel zur verwendung nach einem der vorstehenden Ansprüche zur oralen oder parenteralen Verabreichung an einen Patienten.

7. Mittel zur verwendung nach Anspruch 6, in Form einer wässerigen Injektionslösung.

8. Mittel zur verwendung nach Anspruch 6 oder 7, **dadurch gekennzeichnet, dass** es Ectoin und/oder Hydroxyectoin und/oder ein pharmazeutisch annehmbares Salz davon in einer Menge von 10 bis 10000 mg, vorzugsweise 20 bis 1000 mg und besonders bevorzugt 50 bis 500 mg enthält.

## Claims

1. Agent for use for prophylactic or therapeutic treatment of vascular leaks, **characterised in that** it contains ectoine and/or hydroxyectoine and/or a pharmaceutically acceptable salt thereof as sole active ingredient/sole active ingredients.

2. An agent for use according to claim 1 for the treatment of vasculatis and in particular VLS, SIRS and ARDS (vascular leak syndrome, systemic inflammatory response syndrome, acute respiratory distress syndrome).

3. An agent for use according to one of the preceding claims for the prophylaxis or treatment of forms of vascular leaks, triggered by toxins or drugs.

4. An agent for use according to claim 3 for the inhibition of VLS induced by interleukin-2.

5. An agent for use according to claim 1 or claim 2 for the treatment of VLS triggered by haemorrhagic fevers.

6. An agent for use according to one of the preceding claims for the oral or parenteral administration to a patient.

7. An agent for use according to claim 6 in the form of an aqueous injection solution.

8. An agent for use according to claim 6 or claim 7 **characterised in that** it contains ectoine and/or hydroxyectoine and/or a pharmaceutically acceptable salt thereof in an amount of 10 to 10,000 mg, preferably 20 to 1000 mg and particularly preferably 50 to 500 mg.

## Revendications

1. Moyen pour utilisation pour le traitement prophylactique ou thérapeutique de fuites vasculaires, **caractérisé en ce qu'**il comprend de l'ectoïne et/ou de l'hydroxyectoïne et/ou un sel pharmaceutiquement acceptable de celui-ci comme agent actif unique/agents actifs uniques.

2. Moyen pour utilisation selon la revendication 1 pour le traitement d'une vasculite et en particulier de VLS, SIRS, ARDS (syndrome de fuite vasculaire, syndrome de réponse inflammatoire systémique, syndrome de détresse respiratoire aiguë).

3. Moyen pour utilisation selon l'une des revendications précédentes pour la prophylaxie ou le traitement de formes déclenchées par des toxines ou des médicaments de fuites vasculaires.

4. Moyen pour utilisation selon la revendication 3 pour l'inhibition du VLS induit par l'interleukine-2.

5. Moyen pour utilisation selon la revendication 2 pour le traitement du VLS déclenché par des fièvres hémorragiques.

6. Moyen pour utilisation selon l'une des revendications précédentes pour l'administration orale ou parentérale à un patient.

7. Moyen pour utilisation selon la revendication 6, sous la forme d'une solution d'injection aqueuse.

8. Moyen pour utilisation selon la revendication 6 ou 7, **caractérisé en ce qu'**il contient de l'hectoïne et/ou de l'hydroxyectoïne et/ou un sel pharmaceutiquement acceptable de ceux-ci en une quantité de 10 à 10000 mg, de préférence de 20 à 1000 mg et selon une préférence particulière de 50 à 500 mg.
